(19) Europäisches Patentamt
European Patent Office
Office européen des brevets

(11) Publication number: 0 227 078 A1

(12) # EUROPEAN PATENT APPLICATION

(21) Application number: 86117813.5

(51) Int. Cl.4: C12P 7/40 , C12P 41/00

(22) Date of filing: 19.12.86

(30) Priority: 20.12.85 US 811260
17.03.86 US 840280

(43) Date of publication of application:
01.07.87 Bulletin 87/27

(84) Designated Contracting States:
AT BE CH DE ES FR GB GR IT LI LU NL SE

(71) Applicant: WISCONSIN ALUMNI RESEARCH FOUNDATION
Post Office Box 7365 614 North Walnut
Madison, WI 53707(US)

(72) Inventor: Sih, Charles J.
6322 Landfall Drive
Madison Wisconsin 53705(US)

(74) Representative: Barz, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

(54) **Process for preparing (S)-alpha-methylarylacetic acids.**

(57) The invention relates to a process for preparing (S)-alpha-methylarylacetic acids from mixtures, such as racemic mixtures, of (R)-and (S)-alpha-methylarylacetic acid esters by enantiospecific hydrolysis using extracellular lipases of microbial origin (EC 3.1.1.3). Preferably S-2-(6-methoxy-2-naphthyl)-alpha-methylacetic acid is prepared (S-Naproxen).

EP 0 227 078 A1

# Process for Preparing (S)-α-Methylarylacetic Acids

The present invention relates to processes for producing chiral α-methylarylacetic acids. More specifically, it relates to a process for preparing (S)-α-methylarylacetic acids from mixtures, such as racemic mixtures, of ( R)-and (S)-α-methylarylacetic acid esters by enantiospecific hydrolysis using extracellular lipases of microbial origin (EC 3.1.1.3).

A number of α-methylarylacetic acids (2-arylpropionic acids) are known as antiinflammatory agents: among the best known being ibuprofen, flurbiprofen, ketoprofen, and suprofen (all of which are substituted α-methylbenzeneacetic acids), and naproxen (a substituted α-methylnaphthaleneacetic acid). As is well known, the α-methylarylacetic acid molecule is chiral at the α-carbon atom and, therefore, exists in two stereoisomeric forms: the R-and S-forms (these forms are named by application of the "Sequence Rule", see J. Org. Chem., 35, 2863-7 (1970)). The S-enantiomers of these α-methylarylacetic acids generally possess greater antiinflammatory activity than the R-enantiomers ["Non-steroidal Antiinflammatory Drugs", J.G. Lombardino (ed.), John Wiley & Sons, New York, 1985, p. 303]. For example, the S-enantiomer of 6-methoxy-α-methyl-2-naphthaleneacetic acid has 28 times greater antiinflammatory activity than the R-enantiomer [I.T. Harrison et al., J. Med. Chem., 13, 203 (1970)]. Hence, the S-enantiomer alone is used as the antiinflammatory drug naproxen (USAN and the USP Dictionary of Drug Names, 1986, p. 222).

The chemical synthesis of 6-methoxy-α-methyl-2-naphthaleneacetic acid [I.T. Harrison et al., J. Med. Chem., 13, 203 (1970)] leads to a racemic mixture of R-and S-enantiomers. Hence, resolution methods have to be employed to obtain the separate enantiomers from the racemic mixture. These resolution methods are, however, cumbersome and expensive. Generally, the chemical resolution methods entail the selective stoichiometric crystallization of a diastereomeric salt by the use of an expensive amine such as cinchonidine [P. Wirth et al., Ger. Pat. (Offen.) 2,319,245 (1973); U.S. Pats. 3,787,580; 3,651,106; 3,906,038] or dehydroabietylamine acetate [British Pat. 1,426,186 (1976)], or the use of a water soluble amine such as glucamine, which is difficult to recover [E. Felder et al., U.K. Pat. Appl. 2025968A (1980)]. Naproxen was also prepared by the chemical resolution of a precursor using the expensive and less-available (1)-10-camphorsulfonic acid [G.I. Tsuchihashi, Tet. Lett., 5427 (1982)]. Alternatively, an intact microorganism was used for the partial resolution of (±)-6-methoxy-α-methyl-2-naphthaleneacetic acid methyl ester (S. Iriuchijima and A. Keiyu, Agri. Biol. Chem., 45, 1389 (1981)]. Unfortunately, the rate of conversion was very slow (16.3% of the substrate was converted), because the intracellular enzyme concentration was low and the amount of dried cells (400 mg) exceeded the amount of (±)-ester substrate (160 mg). Consequently, this process failed to achieve the desired objective.

Broadly, this invention comprises a process for preparing an (S)-α-methylarylacetic acid from a substrate comprising a mixture, such as a racemic mixture, of (R )-and (S)-α-methylarylacetic acid esters by enantiospecific hydrolysis using an extracellular lipase of microbial origin (EC 3.1.1.3).

The α-methylarylacetic acid esters which are the substrates for the enantiospecific hydrolysis are of the general formula:

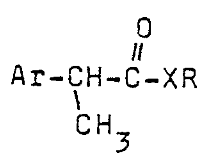

wherein:

Ar is an optionally substituted aryl group;
X is oxygen or sulfur; and
R is an optionally substituted alkyl group.

Ar is preferably a monocyclic, polycyclic, or condensed polycyclic aromatic or heteroaromatic group having up to 12, preferably 6 to 12, carbon atoms in the aromatic system, such as phenyl, biphenyl, naphthyl, thienyl, and pyrrolyl. The aromatic group is optionally substituted with one or more nitro, halo, hydroxy, $C_{1-4}$alkyl, $C_{3-6}$ cycloalkyl, benzyl, $C_{1-4}$ alkoxy, $C_{1-4}$alkylthio, $C_{1-4}$ haloalkyl, $C_{1-4}$ haloalkoxy, phenoxy, thenoyl, and benzoyl groups.

Specific examples of aryl groups, Ar, suitable for the purposes of the present invention are phenyl, 4-benzoylphenyl, 4-isobutylphenyl, 4-(2-thenoyl)phenyl, 3-fluorobiphenyl, 6-methoxy-2-naphthyl, 5-halo-6-methoxy-2-naphthyl, 6-hydroxy-2-naphthyl, and 5-halo-6-hydroxy-2-naphthyl.

X is preferably oxygen.

R is preferably a straight, branched, or cyclic alkyl group having from 1 to 12 carbon atoms, optionally substituted with phenyl or one or more electron-withdrawing substituents, for example halo, nitro, cyano, hydroxy, $C_{1-4}$ alkoxy, $C_{1-4}$ alkylthio, or -C(O)R' wherein R' is $C_{1-4}$ alkyl, $C_{3-6}$ cycloalkyl, hydroxy, $C_{1-4}$ alkoxy, $C_{3-6}$ cycloalkoxy, phenoxy, benzyloxy, $NR^2R^3$ [in which $R^2$ and $R^3$ are independently H, $C_{1-4}$ alkyl, $C_{3-6}$ cycloalkyl, or jointly form a 5-or 6-membered ring together with the nitrogen, the ring optionally including a hetero group selected from O, NH, or N-($C_{1-4}$ alkyl)], or -OM wherein M is an alkali metal.

The electron-withdrawing substituents if present are preferably at the α-or β-position of the R group, to the extent consistent with the stability of the group. Esters in which the R groups contain electron-withdrawing substituents are referred to as activated esters, since they generally hydrolyse more rapidly than those where the R group is not so substituted.

Specific examples of alkyl groups, R, are methyl, ethyl, butyl, hexyl, octyl, dodecyl, benzyl, 2-chloroethyl, 2,2,2-trichloroethyl, 2-fluoroethyl, 2,2,2-trifluoroethyl, 2-bromoethyl, cyanomethyl, 2-nitropropyl, carboethoxymethyl, methoxymethyl, 2-hydroxy-1,2-dimethoxycarbonylethyl, 2-hydroxy-1,2-dicarboxyethyl, 2-hydroxy-1,2-diethoxycarbonylethyl, etc.

The process of the invention comprises subjecting the substrate comprising the mixture of (R)-and (S)-α-methylarylacetic acid esters to the hydrolytic enzymatic action of an extracellular microbial lipase (EC 3.1.1.3) and recovering the desired (S)-α-methylarylacetic acid.

If the resulting (S)-α-methylarylacetic acid is a precursor of a drug such as naproxen, for example (S)-5-halo-6-methoxy-α-methyl-2-naphthaleneacetic acid, (S)-6-hydroxy-α-methyl-2-naphthaleneacetic acid or (S)-5-halo-6-hydroxy-α-methyl-2-naphthaleneacetic acid, such precursor can be converted to naproxen by methods described in European Published Application 95901 (1983).

The esters of the (R)-and (S)-α-methylarylacetic acids which are to be resolved can be prepared by conventional methods (see, e.g., I.T. Harrison and S. Harrison in "Compendium of Organic Synthetic Methods", Chapter 8, Wiley, N.Y., 1971, page 271). Indeed, conventional (non-enantiospecific) syntheses of α-methylarylacetic acids and their esters typically produce racemic, or approximately racemic, mixtures of the R-and S-isomers, so that the substrates for the enantiospecific hydrolysis of this invention are readily available.

It has been found that extracellular microbial lipases are capable of functioning to catalyze enantiospecific hydrolysis. Particularly suitable are those extracellular lipases derived from the microorganisms of the genera Candida, Rhizopus, Mucor, Aspergillus, Penicillium, Pseudomonas, Chromobacterium, and Geotrichium. Particularly preferred is the lipase of Candida cylindracea [N. Tomizuka et al., Agri. Biol. Chem., 30, 576 (1966)].

Extracellular microbial lipases are well known and many of these are available commercially [see, e.g. M. Iwai and Y. Tsujisaka, p. 443, and M. Sugiura, p. 505, in "Lipases," ed. B. Borgström and H.L. Brockman, Elsevier, N.Y., 1984]. For example, they are used industrially for the transesterification of fats and were incorporated in laundry detergents for removal of oily contaminants. One outstanding feature of these lipases that distinguishes them from intact microorganisms is that they can tolerate high substrate and product concentrations. For example, no marked substrate and product inhibition were noted. Hence, these enzymatic hydrolytic reactions can be carried out in high concentrations (0.1-5 M) with an usually high degree of enantiospecificity. Moreover, they are remarkably stable under the described reaction conditions, so that they may be recovered from reaction media (e.g. by filtration on a membrane filter or similar methods) and reused.

The microorganisms producing these lipases may be grown on a liquid nutritional soil according to conventional procedures, for example, by inoculating the microorganism into a sterilized liquid culture medium and causing to grow on an alternating shaker at between 20°C and 40°C for a period of 1-3 days.

Suitable lipase concentrations are those conventional in the art, and are largely determined by experiment based on the desired rate of conversion compared to the lipase cost. For the Candida cylindracea lipase, which has a molecular weight of approximately 100,000, suitable concentrations are about $10^{-5}$M to $10^{-3}$ M, typically about $10^{-4}$M or 10 mg/mL of pure lipase.

The mixture of (R)-and (S)-α-methylarylacetic acid esters constituting the substrate may be added in solid or liquid forms at concentrations of 0.1-5 M, typically 1-2 M, to a liquid medium containing the lipase to effect the enantiospecific hydrolysis. The liquid medium can be water, the same culture broth of the microorganism, or its extracts or concentrates, or the suspensions of the microorganism cells. Alternatively, the substrate can be dissolved in a suitable organic solvent such as carbon tetrachloride, cyclohexane, carbon disulfide, or hexane, as long as the solvent does not denature the lipase. In addition, the substrate may be emulsified by the use of emulsifying agents such as polyvinyl alcohol or propylene glycol. Of course, the time and temperature and pressure conditions for the contact of the substrate with the lipase

are interdependent, as will be apparent to those skilled in the art. Generally, at atmospheric pressure, the temperature can range from about 10°C to about 40°C, and is preferably at the upper end of that range - (e.g. 25-40°C) for maximum conversion rate. The pH of the medium can range from about 3 to about 8, typically from about 4 to about 7, and is preferably maintained relatively constant, e.g. by addition of acid or base or through the use of a buffer solution such as phosphate buffer. The time for the reaction is typically between a few hours and a few weeks, say 4 hours to 3 weeks, and more typically is between about 2 and 7 days. This time can be substantially varied by variation of the temperature and pressure of the reaction, and by the concentration of substrate and lipase, as well as by the nature of the substrate and lipase themselves, and optimization of such conditions may be performed by techniques known to the art.

Following the enantiospecific hydrolysis reaction, the α-methylarylacetic acid enriched in the S-form and the unreacted ester enriched in the R-form may be extracted from the reaction mixture by using water-immiscible organic solvents such as ethyl acetate, methylene chloride, ethyl ether and toluene and the like. Subsequently, the acid enriched in the S-form and the ester enriched in the R-form can be separated by extraction or chromatography.

Alternatively, the α-methylarylacetic acid enriched in the S-form may be isolated by extraction using aqueous alkaline solutions such as aqueous solutions of sodium hydroxide or potassium hydroxide.

The separation of the S-acid and the R-ester may also be accomplished by centrifuging or filtering the reaction mixture to isolate the solid which is the R -ester. Acidifying the supernatant or the filtrate will give the desired S-acid.

It will be obvious to those skilled in the art that the process of this invention as set forth hereinbefore can be modified and perhaps improved by various means.

For example, the process may be made continuous wherein the lipase is immobilized (e.g. on watersoluble or water-insoluble polymers, inorganic materials such as diatomaceous earth, etc.) by conventional techniques [see, e.g., "Immobilized Enzymes", M.D. Trevan, Wiley, N.Y., 1980] and recycled several times to reduce cost; the R-ester can be recovered, racemized [see, e.g., J. Kenyon and D.P. Young, J. Chem. Soc., 216 (1940)] and reused; or the substrate can be exposed to the lipase as a microcrystalline powder to obtain better dispersion. Furthermore, it may be possible to dissolve the substrate and a racemization agent in a suitable solvent so only the ester will be continuously racemized in situ without cleaving the ester grouping. In such case, this process would be tantamount to an asymmetric synthesis. Also, activators and stabilizers of the lipase, such as surfactants (e.g. bile acids, phospholipids) or emulsifiers, in low concentration, [see, e.g., N. Tomizuka et al., Agri. Biol. Chem., 30, 576 (1966)] may be introduced to the mixture, or activated ester substrates [Bodansky et al., Peptide Synthesis, 2nd Ed., Wiley, N.Y., 1976, pp. 99-108] may be used to enhance the rate of conversion. In addition, active site directed mutagenesis or chemical modification of the lipase may be used to prepare lipases with improved catalytic efficiency, $V_{max}/K_m$, ["Enzymatic Reaction Mechanisms", C. Walsh, Freeman, N.Y., 1979, p. 35] and/or stability.

The following examples are presented to illustrate this invention and are not to be considered as limiting the scope of the appended claims.

## Example 1

To a suspension of Candida cylindracea lipase (100 mg) (Sigma L1754 Type VII, 500 units/mg solid) in 1 mL 0.2M phosphate buffer, pH 8.0, were added 244 mg (±)-6-methoxy-α-methyl-2-naphthaleneacetic acid methyl ester as a fine powder, giving a 1 M suspension of the racemic ester substrate, and 100 mg polyvinyl alcohol (MW 14,000). The reaction mixture was stirred with a magnetic stirrer for 6 days at 24°C. The contents were then acidified with HCl and exhaustively extracted with ethyl acetate three times. The combined organic extract was dried over sodium sulfate and was then evaporated to dryness. The residue was suspended in 5% aqueous $NaHCO_3$ and extracted with hexane to obtain unreacted (R)-6-methoxy-α-methyl-2-naphthaleneacetic acid methyl ester (128 mg), $[\alpha]_D^{23}$ = -41.35° (c = 5.34, $CHCl_3$). Acidification of the aqueous layer with HCl to pH 2.0, followed by extraction with dichloromethane, gave 58 mg (S)-6-methoxy-α-methyl-2-naphthaleneacetic acid, $[\alpha]_D^{23}$ = +65.0° (c = 1.64, $CHCl_3$).

## Examples 2-7

The general procedure of Example 1 was repeated except that the different esters of (±)-6-methoxy-α-methyl-2-naphthaleneacetic acid tabulated below were used as substrates. In each case, (S)-6-methoxy-α-methyl-2-naphthaleneacetic acid was obtained in good yield, with the optical activity shown.

| Example | Ester | $[\alpha]_D^{23}$ |
|---|---|---|
| 2 | ethyl | +64.2° (c = 0.59, $CHCl_3$) |
| 3 | n-butyl | +64.3° (c = 0.6, $CHCl_3$) |
| 4 | n-hexyl | +64.7° (c = 1.2, $CHCl_3$) |
| 5 | n-octyl | +63.6° (c = 1.8, $CHCl_3$) |
| 6 | n-dodecyl | +59.8° (c = 1.3, $CHCl_3$) |
| 7 | benzyl | +61.6° (c = 0.9, $CHCl_3$) |

Example 8

The general procedure of Example 1 was repeated except that Candida cylindracea lipase immobilized on celite (as described by Y. Kimura et al., Eur. J. Appl. Microbiol. Biotechnol. , 17, 107 (1983)) was used as the lipase to obtain ( S)-6-methoxy-α-methyl-2-naphthaleneacetic acid in good yield, $[\alpha]_D^{23}$ = +60.18° (c = 1.1, $CHCl_3$).

Example 9

The general procedure of Example 1 was repeated except that Candida cylindracea lipase (Sigma) immobilized on acrylic beads [K. Laumen et al., Tet. Lett., 407 (1985)] was used as the lipase to obtain (S)-6-methoxy-α-methyl-2-naphthaleneacetic acid, $[\alpha]_D^{25}$ = +63.8° (c = 1.2, $CHCl_3$).

Example 10

The general procedure of Example 1 was repeated except that 2500 units Chromobacterium viscosum lipase (Type XII, Sigma) was used as the lipase to obtain optically active 6-methoxy-α-methyl-2-naphthaleneacetic acid.

Example 11

The general procedure of Example 1 was repeated except that 10 mg Pseudomonas lipo-protein Lipaic 80 (Amano, 800 u/gm) was used as the lipase to obtain optically active 6-methoxy-α-methyl-2-naphthaleneacetic acid.

Example 12

The general procedure of Example 1 was repeated except that 10 mg purified Geotrichium candidum - (ATCC) 34614) lipase [Y. Tsujisaka et al., Agr. Biol. Chem., 37 , 1457 (1973)] was used as the lipase to obtain optically active 6-methoxy-α-methyl-2-naphthaleneacetic acid.

Example 13

The general procedure of Example 1 was repeated except that 200 mg crude lipase of Penicillium cyclopium ATCC 34613 [M. Iwai et al., Agr. Biol. Chem., 39, 1063 (1975)] was used as the lipase to obtain optically active 6-methoxy-α-methyl-2-naphthaleneacetic acid.

Example 14

To 10 mg purified Candida cylindracea lipase (1100 units/mg) [N. Tomizuka et al., Agr. Biol. Chem., 30, 576 (1966)] in 1 mL of 0.2M phosphate buffer, pH 8.0, was added 244 mg (±)-6-methoxy-α-methyl-2-naphthaleneacetic acid methyl ester as a fine powder, giving a 1 M suspension. The resulting suspension was gently stirred with a magnetic stirrer for 5 days at 22°C. The reaction mixture was then centrifuged for 5 min at 1000 x g. The precipitate was washed at once with 0.2 M phosphate buffer, pH 8.0, and again centrifuged to collect the unreacted water-insoluble (R)-6-methoxy-α-methyl-2-naphthaleneacetic acid methyl ester (94 mg), $[\alpha]_D^{23}$ = -72.39° (c = 6.98, $CHCl_3$). The supernatant and the washings were combined and acidified to pH 2.5 with 3N HCl and the precipitate was collected by centrifugation to yield 96 mg (S)-6-methoxy-α-methyl-2-naphthaleneacetic acid, $[\alpha]_D^{23}$ = +68.87° (c = 5.22, $CHCl_3$).

Example 15

To 50 mg crude Candida cylindracea lipase (Sigma L1754 Type VII, 500 units/mg solid) in 1 mL 0.2 M phosphate buffer, pH 8.0, were added 292 mg (±)-6-methoxy-α-methyl-2-naphthaleneacetic acid 2-chloroethyl ester as a fine powder, giving a 1 M suspension 1 x $10^{-3}$M mercaptoethanol, and 10 mg polyvinyl alcohol. The resulting suspension was gently stirred with a magnetic stirrer for 42 hours at 22°C. The reaction mixture was then centrifuged for 5 min at 1000 x g and the precipitate was washed with 0.2M phosphate buffer, pH 8.0, and again centrifuged to collect the unreacted water-insoluble (R)-6-methoxy-α-methyl-2-naphthaleneacetic acid 2-chloroethyl ester (140 mg), $[\alpha]_D^{23}$ = -20.5° (c = 4.96, $CHCl_3$). The supernatant and the washings were combined and acidified to pH 2.0 with 3N HCl and the precipitate was collected by filtration to afford 92 mg of (S)-6-methoxy-α-methyl-2-naphthaleneacetic acid, $[\alpha]_D^{23}$ = +64.2° (c = 3.49, $CHCl_3$).

Example 16

To 10 mg Candida cylindracea lipase (Meito Sangyo Lipase OF-360, 360,000 u/g) in 1 mL 0.2 M phosphate buffer, pH 8.0, were added 292 mg (±)-6-methoxy-α-methyl-2-naphthaleneacetic acid 2-chloroethyl ester as a fine powder, giving a 1 M suspension, 1 x $10^{-3}$M mercaptoethanol, and 10 mg polyvinyl alcohol. The resulting suspension was gently stirred with a magnetic stirrer for 48 hours at 22°C. The reaction mixture was then filtered, and the precipitate was washed with 0.2 M phosphate buffer, pH 8.0. The solid consisted of unreacted (R)-6-methoxy-α-methyl-2-naphthaleneacetic acid 2-chloroethyl ester (120 mg), $[\alpha]_D^{25}$ = -16.51° (c = 7.73, $CHCl_3$). The filtrate and the washings were combined and acidified to pH 2.0 with 3N HCl and the precipitate was collected by filtration to give 66 mg (S)-6-methoxy-α-methyl-2-naphthaleneacetic acid, $[\alpha]_D^{25}$ = +61.18° (c = 3.3, $CHCl_3$).

Examples 17-24

The general procedure of Example 16 was repeated except that the different esters of (±)-6-methoxy-α-methyl-2-naphthaleneacetic acid were used as substrates to obtain (S)-6-methoxy-α-methyl-2-naphthaleneacetic acid.

| Example | Ester | $[\alpha]_D^{23}$ |
| --- | --- | --- |
| 17 | 2,2,2-trichloroethyl | +67.0° (c = 1.25, $CHCl_3$) |
| 18 | cyanomethyl | +67.07° (c = 2.59, $CHCl_3$) |
| 19 | 2-nitropropyl | +61.8° (c = 5.2, $CHCl_3$) |
| 20 | 2-bromoethyl | +60.8° (c = 4.1, $CHCl_3$) |
| 21 | carboethoxymethyl | +62.07° (c = 3.18, $CHCl_3$) |
| 22 | methoxymethyl | +63.28° (c = 2.17, $CHCl_3$) |
| 23 | 2-fluoroethyl | +62.81° (c = 3.84, $CHCl_3$) |
| 24 | 2,2,2-trifluoroethyl | +61.87° (c = 4.13, $CHCl_3$) |

2255Y 25810-FF

Example 25

To 100 mg crude Candida cylindracea lipase (Sigma L1754 Type VII, 500 units/mg solid) in 4 mL 0.2 M phosphate buffer, pH 7.0, was added 200 mg (±)-α-methyl-4-(2-thienylcarbonyl)benzeneacetic acid methyl ester (suprofen methyl ester). The resulting suspension was gently stirred with a magnetic stirrer for 48 hours at 22°C. The reaction mixture was acidified to pH 1.0 with 1N HCl and exhaustively extracted with ethyl acetate three times. The combined organic extract was dried over sodium sulfate and was then evaporated to dryness. The residue was chromatographed over a silica gel (MN Kieselgel 60) column (0.8 x 15 cm). Elution of the column with a solvent system consisting of ethyl acetate-hexane (1:5) gave unreacted (R)-α-methyl-4-(2-thienylcarbonyl)benzeneacetic acid methyl ester, $[\alpha]_D^{25}$ = -54.7° (c = 3.9, $CHCl_3$), ee > 0.90, and (S)-α-methyl-4-(2-thienylcarbonyl)benzeneacetic acid, $[\alpha]_D^{25}$ = +43.5° (c = 2.1, $CHCl_3$), ee > 0.95. The optical purity expressed as enantiomeric excess (ee) is determined by proton magnetic resonance spectroscopy of the methyl ester in the presence of the chiral lanthanide shift reagent, $Eu(hfc)_3$.

Example 26

The general procedure of Example 25 was repeated except that 200 mg of (±)-α-methyl-4-(2-methylpropyl)-benzeneacetic acid methyl ester (ibuprofen methyl ester) was used as the substrate. Unreacted (R)-α-methyl-4-(2-methylpropyl)benzeneacetic acid methyl ester, $[\alpha]_D^{25}$ = -45.1° (c = 5.3, $CHCl_3$), ee = 0.70, and (S)-α-methyl-4-(2-methylpropyl)benzeneacetic acid, $[\alpha]_D^{25}$ = +50.44° (c = 2.7, $CHCl_3$), ee = 0.95, were recovered.

Example 27

The general procedure of Example 25 was repeated except that 200 mg of (±)-α-methylbenzeneacetic acid methyl ester was used as the substrate. Unreacted (R)-α-methylbenzeneacetic acid methyl ester, $[\alpha]_D^{25}$ = -67.8° (c = 2.2, $CHCl_3$), ee = 0.80, and (S)-α-methylbenzeneacetic acid, $[\alpha]_D^{25}$ = +27.4° (c = 2.0, $CHCl_3$), ee = 0.45, were recovered.

Example 28

The general procedure of Example 25 was repeated except that 200 mg (±)-2-fluoro-α-methyl-[1,1'-biphenyl]-4-acetic acid methyl ester (flurbiprofen methyl ester) was used as the substrate and 100 mg Candida cylindracea lipase (Meito Sangyo Lipase OF-360, 360,000 u/g) as the lipase. Unreacted (R)-2-fluoro-α-methyl-[1,1'-biphenyl]-4-acetic acid methyl ester, $[\alpha]_D^{25}$ = -21.5° (c = 4.8, $CHCl_3$), ee = 0.41, and (S)-2-fluoro-α-methyl-[1,1'-biphenyl]-4-acetic acid, $[\alpha]_D^{25}$ = +29.7° (c = 2.3, $CHCl_3$), ee = 0.65, were recovered.

Example 29

The general procedure of example 25 was repeated except that 200 mg (±)-3-benzoyl-α-methylbenzene-acetic acid methyl ester (ketoprofen methyl ester) was used as the substrate and 100 mg Candida cylindracea lipase (Meito Sangyo Lipase OF-360, 360,000 u/g) as the lipase. Unreacted (R)-3-benzoyl-α-methylbenzeneacetic acid methyl ester $[\alpha]_D^{25}$ = -43.8° (c = 1.6, $CHCl_3$), ee = 0.60, and (S-3-benzoyl-α-methyl-benzeneacetic acid, $[\alpha]_D^{25}$ = +34.3° (c = 3.5, $CHCl_3$), ee = 0.60, were recovered.

## Claims

1. A process for preparing an ( S)-α-methylarylacetic acid from a substrate comprising a mixture of (R)- and (S)-α-methylarylacetic acid esters which comprises subjecting the substrate to enantiospecific hydrolysis using an extracellular lipase of microbial origin (EC 3.1.1.3) and recovering the desired (S)-α-methylarylacetic acid.

2. The process of Claim 1 wherein the mixture of (R)-and (S)-α-methylarylacetic acid esters is a racemic mixture.

3. The process of Claims 1 or 2 wherein the α-methylarylacetic acid ester has the formula:

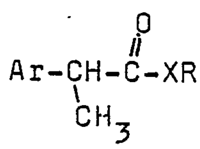

in which
Ar is a monocyclic, polycyclic, or condensed polycyclic aromatic or heteroaromatic group having up to 12 carbon atoms in the aromatic system, optionally substituted with one or more nitro, halo, hydroxy, $C_{1-4}$alkyl, $C_{3-6}$ cycloalkyl, benzyl, $C_{1-4}$ alkoxy, $C_{1-4}$alkylthio, $C_{1-4}$ haloalkyl, $C_{1-4}$ haloalkoxy, phenoxy, thenoyl, and benzoyl groups;
X is oxygen or sulfur; and
R is a straight, branched, or cyclic alkyl group having from 1 to 12 carbon atoms, optionally substituted with phenyl or one or more electron-withdrawing substituents.

4. The process of Claim 3 wherein Ar is selected from phenyl, 4-benzoylphenyl, 4-isobutylphenyl, 4-(2-thenoyl)-phenyl, 3-fluorobiphenyl, 6-methoxy-2-naphthyl, 5-halo-6-methoxy-2-naphthyl, 6-hydroxy-2-naphthyl and 5-halo-6-hydroxy-2-naphthyl.

5. The process of Claim 3 or 4 wherein X is oxygen.

6. The process of any one of Claims 3 through 5 wherein the electron-withdrawing substituents are selected from halo, nitro, cyano, hydroxy, $C_{1-4}$ alkylthio, $C_{1-4}$ alkoxy, or -C(O)R' [wherein R' is $C_{1-4}$ alkyl, $C_{3-6}$cycloalkyl, hydroxy, $C_{1-4}$alkoxy, $C_{3-6}$ cycloalkoxy, phenoxy, benzyloxy, $NR^2R^3$ (in which $R^2$ and $R^3$ are independently H, $C_{1-4}$alkyl, $C_{3-6}$ cycloalkyl, or jointly form a 5-or 6-membered ring together with the nitrogen, the ring optionally including a hetero group selected from O, NH, or N-($C_{1-4}$ alkyl)), or -OM wherein M is an alkali metal], and the electron-withdrawing substituents if present are preferably at the α-and β-position of the R group.

7. The process of any one of the preceding Claims wherein the substrate is an activated ester.

8. The process of any one of the preceding Claims wherein an activator for the lipase is present.

9. The process of any one of the preceding Claims wherein the lipase is immobilized.

10. The process of any one of the preceding Claims wherein the lipase is derived from microorganisms selected from the genera consisting of Candida, Rhizopus, Mucor, Aspergillus, Penicillium, Pseudomonas, Chromobacterium, and Geotrichium .

11. The process of Claim 10 wherein the lipase is derived from Candida cylindracea.

12. The process of Claim 11 wherein the substrate is selected from (±)-α-methyl-4-(2-methylpropyl)-benzeneacetic acid methyl ester; (±)-α-methyl-4-(2-methylpropyl)benzeneacetic acid 2-chloroethyl ester; (±)-2-fluoro-α-methyl-[1,1'-biphenyl]-4-acetic acid methyl ester; (±)-2-fluoro-α-methyl-[1,1'-biphenyl]-4-acetic

acid 2-chloroethyl ester; (±)-3-benzoyl-α-methylbenzeneacetic acid methyl ester; (±)-3-benzoyl-α-methylbenzeneacetic acid 2-chloroethyl ester; (±)-α-methyl-4-(2-thienylcarbonyl)benzeneacetic acid methyl ester; and (±)-α-methyl-4-(2-thienylcarbonyl)benzeneacetic acid 2-chloroethyl ester.

13. A process for preparing (S)-6-methoxy-α-methyl-2-naphthaleneacetic acid which comprises subjecting a substrate comprising a mixture of (R)-and (S)-6-methoxy-α-methyl-2-naphthaleneacetic acid esters to the hydrolytic enzymatic action of an extracellular microbial lipase, separating the unreacted (R)-6-methoxy-α-methyl-2-naphthaleneacetic acid ester, and recovering the desired (S)-6-methoxy-α-methyl-2-naphthaleneacetic acid.

14. The process of Claim 13 wherein the substrate is (±)-6-methoxy-α-methyl-2-naphthaleneacetic acid methyl ester or (±)-6-methoxy-α-methyl-2-naphthaleneacetic acid 2-chloroethyl ester.

15. The process of Claims 13 or 14 wherein the lipase is derived from Candida cylindracea and is, preferably, Meito Sangyo Lipase OF-360.

European Patent Office

# EUROPEAN SEARCH REPORT

Application number

EP 86 11 7813

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | AGRICULTURAL & BIOLOGICAL CHEMISTRY, vol. 45, no. 6, June 1981, pages 1389-1392, Tokyo, JP; S. IRIUCHIJIMA et al.: "Asymmetric hydrolysis of (+/-)-alpha-substituted carboxylic acid esters with microorganisms" * Page 1390, figure 1, formula 12; page 1391, right-hand column, paragraph 6 * | 1-6,10,13,14 | C 12 P 7/40<br>C 12 P 41/00 |
| A | EP-A-0 153 474 (BOEHRINGER MANNHEIM) * Claims * | 1-6,13,14 | |
| P,X | EP-A-0 196 625 (STAUFFER) (Publ. 08-10-1986) * Claims; page 3, paragraphs 2,3 * | 1-11 | TECHNICAL FIELDS SEARCHED (Int. Cl.4) |
| T | TETRAHEDRON LETTERS, vol. 27, no. 16, 1986, pages 1763-1766, Pergamon Press Ltd, GB; Q.-M. GU et al.: "A facile enzymatic resolution process for the preparation of (+)-S-2-(6-methoxy-2-naphthyl) propionic acid (naproxen)" * Whole document * | 1-15 | C 12 P<br>C 12 R |

The present search report has been drawn up for all claims

| Place of search | Date of completion of the search | Examiner |
|---|---|---|
| THE HAGUE | 16-03-1987 | COUCKUYT D.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82